(19) Europäisches Patentamt
European Patent Office
Office européen des brevets

(11) EP 0 764 628 B1

(12) EUROPÄISCHE PATENTSCHRIFT

(45) Veröffentlichungstag und Bekanntmachung des Hinweises auf die Patenterteilung:
**14.03.2001 Patentblatt 2001/11**

(51) Int Cl.[7]: **C07C 68/06**, C07C 69/96

(21) Anmeldenummer: **96810475.2**

(22) Anmeldetag: **19.07.1996**

# (54) Pyrokohlensäurediester und deren Herstellung sowie Verwendung

Diesters of pyrocarbonic acid, their preparation and their use

Diesters de l'acide pyrocarbonique, leur préparation et leur utilisation

(84) Benannte Vertragsstaaten:
**CH DE FR GB LI**

(30) Priorität: **28.07.1995 CH 222295**

(43) Veröffentlichungstag der Anmeldung:
**26.03.1997 Patentblatt 1997/13**

(73) Patentinhaber: **Ciba Specialty Chemicals Holding Inc.**
**4057 Basel (CH)**

(72) Erfinder:
- **Zambounis, John**
  **4056 Basel (CH)**
- **Hall-Goulle, Véronique**
  **3012 Bern (CH)**

(56) Entgegenhaltungen:
**EP-A- 0 468 404**

- **PATENT ABSTRACTS OF JAPAN vol. 18, no. 482 (C-1247), 8.September 1994 & JP-A-06 157422 (MITSUBISHI GAS CHEM CO INC), 3.Juni 1994,**
- **DATABASE WPI Week 9401 Derwent Publications Ltd., London, GB; AN 94-002263 XP002016311 & JP-A-05 310 646 (DAIICHI KAGAKU YAKUSHIN KK) , 22.November 1993**
- **DATABASE CROSSFIRE Beilsteins Informationssysteme GMBH,Frankfurt DE XP002016310 & J.ORG.CHEM. USSR(ENGL.TRANSL.), Bd. 14, 1978, Seite 674 POZDNEV ET AL.:**
- **DATABASE CROSSFIRE Beilstein Informationssysteme GMBH,Frankfurt DE XP002016319 & J.ORG.CHEM. USSR(ENGL.TRANSL.), Bd. 7, Nr. 8, 1971, Seiten 1719-1722, POZDNEV V.F.:**
- **DATABASE CROSSFIRE Beilstein Informationssysteme GMBH,Frankfurt DE XP002016327 & J.ORG.CHEM, Bd. 36, 1971, Seiten 1180-1183, DEAN C.S. ET AL.:**
- **DATABASE CROSSFIRE Beilstein Informationssysteme GMBH,Frankfurt DE XP002016328 & J.GEN.CHEM.USSR(ENGL.TRANSL.), Bd. 49, 1979, Seiten 935-939, FOMIN ET AL.:**
- **DATABASE CROSSFIRE Beilstien Informationssysteme GMBH,Frankfurt DE XP002016329 & BULL.ACAD.SCI.USSR DIV.CHEM.SCI.(ENGL.TRANSL), Bd. 28, 1979, Seiten 535-539, TOLMACHEVA G.M. ET AL.:**
- **DATABASE CROSSFIRE Beilstein Informationssysteme GMBH,Frankfurt DE XP002016330 & J.AMER.CHEM.SOC., Bd. 78, 1956, Seiten 174-179, COREY;BURKE:**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 764 628 B1

Printed by Jouve, 75001 PARIS (FR)

## Beschreibung

Diese Erfindung betrifft nach einem verbesserten Verfahren hergestellte neue Pyrokohlensäurediester und deren Verwendung. Die Produkte werden in hoher Ausbeute erhalten und weisen eine sehr hohe Reinheit auf.

Pyrokohlensäurediester selbst sind Wirkstoffe und werden zum Beispiel als Antiseptika für Lebensmittel eingesetzt. Darüber hinaus sind bestimmte Pyrokohlensäurediester wie zum Beispiel Di-tert.-butyl-dicarbonat (DIBOC) wichtige Feinchemikalien, beispielsweise zur Einführung von Schutzgruppen wie Carbonat in Alkoholen, Thiocarbonat in Thiolen oder insbesondere Urethan in Aminen oder Amiden. Solche Gruppen zeichnen sich dadurch aus, dass sie bei Normalbedingungen stabil sind, aber trotzdem zum Beispiel hydrolytisch oder thermisch, unter Rückbildung der ursprünglichen Funktionen gespalten werden können.

Stoffe, welche diese Eigenschaften für technische Zwecke ausnützen, sind aus EP 648 770 und EP 648 817 bekannt. Im Gegensatz zu üblichen aus DIBOC hergestellten tert.-Butyl-urethanen werden in solchen Fällen jedoch präzisere Anforderungen an die Eigenschaften, insbesondere die thermischen Eigenschaften, gestellt. Deshalb besteht der Bedarf nach neuen Pyrokohlensäurediestern als Synthesebausteine.

Angesichts der Wichtigkeit als Lebensmittelzusätze und Feinchemikalien werden an DIBOC und andere Dicarbonate höchste Ansprüche inbezug auf die Reinheit gestellt. Deshalb wurden viele Anstrengungen unternommen, um DIBOC in immer höherer Qualität und Ausbeute herzustellen. Dies ist umso schwieriger, als DIBOC wie in J. Org. Chem. 43, 2410 (1978) angegeben thermisch labil ist.

Zur Herstellung von DIBOC gibt es zwei grundlegend verschiedene Verfahren. Im ersten Verfahren, beschrieben in Org. Synth. 57, 45 (1975) und JP-88/051358, wird tert.-Butyl-carbonat mit Phosgen zu Di-tert.-butyl-tricarbonat umgesetzt, welches anschliessend in Gegenwart eines tertiären Amins (zum Beispiel 1,4-Diazabicyclo[2.2.2]octan) als Katalysator decarboxyliert wird, wobei nach JP-91/356445 bevorzugt noch ein Phasentransferkatalysator zugesetzt wird. In JP-92/310646 wird als tertiäres Amin Pyridin in nicht spezifizierter Menge verwendet. Anstatt von Phosgen kann laut JP-89/186847 auch Thionylchlorid verwendet werden.

Ähnlich dazu offenbart Zhurnal Organicheskoi Khimii 14/4, 730-732 (1978) die Herstellung von 1-Methyl-1-benzylethyl-pyrocarbonat mit Trichloracetylchlorid in Dimethylformamid, wobei die Gesamtausbeute 52,4% beträgt.

Im zweiten, allgemein bevorzugten Verfahren nach Zh. Org. Khim. 15/1, 106 (1975) wird tert.-Butyl-carbonat mit einem Säurechlorid zu einem gemischten Carbonsäureanhydrid umgesetzt, welches mit überschüssigem tert.-Butyl-carbonat in das gewünschte Dicarbonat umgewandelt wird. Dieses Verfahren kann mit dem Ersatz von Carbonsäureanhydriden durch Sulfochloride verbessert werden (CS-247845 und CS-247846). Weitere Massnahmen zur Verbesserung dieses Verfahrens wurden ebenfalls vorgeschlagen, so der Zusatz eines quaternären Ammoniumsalzes (CS-257157), der Gebrauch von Aminen mit aliphatisch gebundenem tertiären Stickstoff, wie beispielsweise Triethylamin, N,N-Dimethylbenzylamin oder N,N,N',N'-Tetramethylethylendiamin (JP-90/103562), eine Konzentration freier Natronlauge unterhalb 10 Mol% (JP-92/194326) oder eine solche freien Natriumalkoholates unterhalb 3 Mol% (JP-92/310648), die Verwendung von Kohlenstoffdioxid unter Druck (JP-92/279301) sowie das salzfreie Waschen des Rohproduktes vor der Destillation (JP-92/306261). JP-06/157422 offenbart eine hohe Ausbeute in kurzer Reaktionszeit, wobei neben einem tertiären Amin auch Dimethylsulfoxid verwendet wird.

Es hat sich jedoch gezeigt, dass alle oben beschriebenen Verfahren trotz Verbesserungen immer noch nicht voll befriedigend sind. So werden hohe Ausbeuten nur bei erhöhten Temperaturen, durch längere Reaktionszeiten oder in Gegenwart grösserer Mengen von polaren hydrophilen Lösungsmitteln wie Tetrahydrofuran, Dimethylsulfoxid oder Dimethylformamid erzielt. Solche polare hydrophile Lösungsmittel, rein oder als Gemische verwendet, lassen sich jedoch - wenn überhaupt - schlecht rezyklieren, und Rückstände können beim Waschen ins Abwasser gelangen. Diese Verfahren sind somit mit grossen ökologischen Problemen verbunden, welche nur mit beträchtlichem Kostenaufwand befriedigend gelöst werden können. Zudem lässt die Qualität der erhaltenen Rohprodukte zu wünschen übrig, so dass eine besonders sorgfältige, zeitraubende fraktionierte Destillation zur Isolierung des Reinproduktes notwendig ist. Wegen der thermischen Empfindlichkeit von DIBOC sollte jedoch eine Destillation wenn möglich ganz vermieden oder mindestens sehr rasch, zum Beispiel im einstufigen Fallfilmverdampfer, durchgeführt werden.

In CS-260076 wird vorgeschlagen, dieses Verfahren in Gegenwart von Pyridin und eines quaternären Ammoniumsalzes durchzuführen. Dies erlaubt zwar eine Reaktion bei Raumtemperatur, jedoch beträgt die Ausbeute lediglich 38,6% d.Th., weshalb zur Verbesserung der Ausbeute eine erhöhte Temperatur von bevorzugt 50°C angegeben wird. Es hat sich jedoch gezeigt, dass dabei die Zersetzung des Produktes eingeleitet wird, und dass letzteres grössere Mengen unreagiertes p-Toluol-sulfochlorid enthält, so dass diese Methode die oben beschriebenen Probleme nicht löst.

EP 468 404 schlägt deshalb vor, zwecks verbesserter Reaktivität Tosylchlorid durch Mesylchlorid zu ersetzen. Mit diesem Verfahren sollen sich beschreibungsgemäss gute Produktqualitäten herstellen lassen, und mit Zusatz von Phasentransferkatalysatoren, aromatischen Aminen oder deren Gemische liesse sich die Ausbeute auch noch erhöhen.

Es zeigt sich jedoch in der Praxis, dass auch letzteres Verfahren mit Problemen behaftet ist. So wurde gefunden, dass eine sehr starke Rührung des Reaktionsgemisches ganz wesentlich ist; praktisch ist dies im Labor nur mit einem speziellen Rührwerk möglich, und beim scale-up sinken die Ausbeuten drastisch. Anscheinend wird diese Schwierigkeit mindestens teilweise durch die physikalischen Eigenschaften von Mesylchlorid verursacht, welches eine Flüssigkeit hoher spezifischer Dichte ist. Mesylchlorid hat weiter einen relativ hohen Dampfdruck und ist sehr ätzend; es hydrolysiert leicht, und reagiert auch mit dem umzusetzenden Alkoholat unter Bildung von Methansulfonsäurealkylestern, welche einen ähnlichen Siedepunkt haben wie die gewünschten Pyrokohlensäurediester, so dass deren Spuren kaum destillativ entfernt werden können. Wegen dieser unerwünschten Nebenreaktion wird Mesylchlorid manchmal auch in leichtem Überschuss verwendet. Letztlich lässt sich die entstehende Methansulfonsäure zwecks Wiederverwertung nicht so leicht aus der wässrigen Lösung isolieren, wie dies wünschbar wäre.

Es wurde nun überraschenderweise gefunden, dass Pyrokohlensäurediester sehr wohl aus Estercarbonat und Tosylchlorid in ausgezeichneten Ausbeute und Reinheit erhalten werden, falls diese Reaktion in Gegenwart eines Ammoniumsalzes und sehr kleiner Mengen Pyridin in einem unpolaren inerten Lösungsmittel durchgeführt wird. Anstatt Tosylchlorid und Pyridin können auch strukturell ähnliche Verbindungen mit vergleichbarem Ergebnis verwendet werden.

Die Pyrokohlensäurediester der Formel (I),

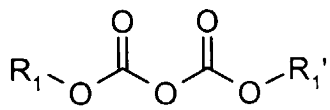 (I)

worin $R_1$ und $R_1'$ unabhängig voneinander unsubstituiertes oder mit einem oder mehreren unter den Reaktionsbedingungen inerten Substituenten substituiertes verzweigtes oder unverzweigtes $C_1$-$C_{24}$-Alkyl, $C_3$-$C_{24}$-Alkenyl, $C_3$-$C_{24}$-Alkinyl, $C_4$-$C_{12}$-Cydoalkyl, $C_4$-$C_{12}$-Cycloalkenyl oder $C_7$-$C_{24}$-Aralkyl bedeuten,
werden durch Umsetzung mindestens eines Estercarbonates der Formel (II),

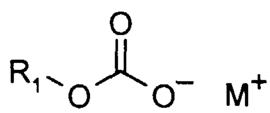 (II)

worin $M^+$ für $Na^+$, $Li^+$, $K^+$ oder $NR_2R_3R_4R_5^+$ steht, und $R_2$ bis $R_5$ unabhängig voneinander Wasserstoff, $C_1$-$C_{18}$-Alkyl, $C_5$-$C_{10}$-Cycloalkyl oder $C_7$-$C_{18}$-Aralkyl bedeuten,
mit 40-50 Mol% eines Sulfochlorides der Formel (IV),

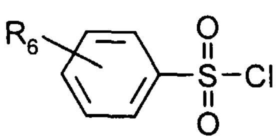 (IV)

worin $R_6$ -H, $-CH_3$, $-CH_2CH_3$, -Cl, -Br, $-OCH_3$ oder $-NO_2$ bedeutet,
in Gegenwart von 0,8-5 Mol% eines Katalysators der Formel (V),

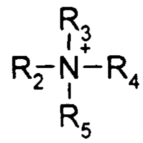 $X^-$ (V)

worin

$R_2$ bis $R_5$ die oben angegebene Bedeutung haben, und
$X^-$ für ein nicht nukleophiles Anion steht,

und geringen Mengen eines heterozyklischen aromatischen Amins in einem unpolaren inerten Lösemittel, erhalten, wobei dieses Verfahren dadurch gekennzeichnet ist, dass die Menge heterozyklisches aromatisches Amin 1-5 Mol% beträgt und die Reaktion bei einer Temperatur zwischen -10°C und +25°C durchgeführt wird,
und alle Molangaben auf 100 Mol% Estercarbonat der Formel (II) bezogen sind.

$C_1$-$C_{18}$-Alkyl ist zum Beispiel Methyl, Ethyl, n-Propyl, Isopropyl, n-Butyl, sec.-Butyl, Isobutyl, tert.-Butyl, n-Pentyl, 2-Pentyl, 3-Pentyl, 2,2-Dimethylpropyl, n-Hexyl, n-Octyl, 1,1,3,3-Tetramethylbutyl, 2-Ethylhexyl, Nonyl, Decyl, Dodecyl, Tetradecyl, Hexadecyl oder Octadecyl. $C_1$-$C_{24}$-Alkyl kann darüberhinaus beispielsweise auch Eicosyl, Heneicosyl, Docosyl oder Tetracosyl bedeuten.

$C_3$-$C_{24}$-Alkenyl ist $C_3$-$C_{24}$-Alkyl, welches ein- oder mehrfach ungesättigt ist, wobei zwei oder mehr Doppelbindungen gegebenenfalls isoliert oder konjugiert sein können, zum Beispiel Allyl, 2-Propen-2-yl, 2-Buten-1-yl, 3-Buten-1-yl, 1,3-Butadien-2-yl, 2-Penten-1-yl, 3-Penten-2-yl, 2-Methyl-1-buten-3-yl, 2-Methyl-3-buten-2-yl, 3-Methyl-2-buten-1-yl, 1,4-Pentadien-3-yl, oder die verschiedenen Isomeren von Hexenyl, Octenyl, Nonenyl, Decenyl, Dodecenyl, Tetradecenyl, Hexadecenyl, Octadecenyl, Eicosenyl, Heneicosenyl, Docosenyl, Tetracosenyl, Hexadienyl, Octadienyl, Nonadienyl, Decadienyl, Dodecadienyl, Tetradecadienyl, Hexadecadienyl, Octadecadienyl, Eicosadienyl, Heneicosadienyl, Docosadienyl oder Tetracosadienyl.

$C_4$-$C_{12}$-Cycloalkyl ist zum Beispiel ein monozyklisches Cycloalkyl, wie beispielsweise Cyclobutyl, Cyclopentyl, Cyclohexyl, Trimethylcyclohexyl oder Menthyl, oder ein poly-zyklisches Cycloalkyl, wie beispielsweise Thujyl, Bornyl, 1-Adamantyl oder 2-Adamantyl.

$C_4$-$C_{12}$-Cycloalkenyl ist $C_4$-$C_{12}$-Cycloalkyl, welches ein- oder mehrfach ungesättigt ist, wobei zwei oder mehr Doppelbindungen gegebenenfalls isoliert oder konjugiert sein können, zum Beispiel 2-Cyclobuten-1-yl, 2-Cyclopenten-1-yl, 2-Cyclohexen-1-yl, 3-Cyclohexen-1-yl, 2,4-Cyclohexadien-1-yl, 1-*p*-Menthen-8-yl, 4(10)-Thujen-10-yl, 2-Norbornen-1-yl, 2,5-Norbornadien-1-yl oder 7,7-Dimethyl-2,4-norcaradien-3-yl.

$C_3$-$C_{24}$-Alkinyl ist $C_3$-$C_{24}$-Alkyl oder $C_3$-$C_{24}$-Alkenyl, welches ein- oder mehrfach doppelt ungesättigt ist, wobei die Dreifachbindungen gegebenenfalls isoliert oder unter sich oder mit Doppelbindungen konjugiert sein können, zum Beispiel 1-Propin-3-yl, 1-Butin-4-yl, 1-Pentin-5-yl, 2-Methyl-3-butin-2-yl, 1,4-Pentadiin-3-yl, 1,3-Pentadiin-5-yl, 1-Hexin-6-yl, cis-3-Methyl-2-penten-4-in-1-yl, trans-3-Methyl-2-penten-4-in-1-yl, 1,3-Hexadiin-5-yl, 1-Octin-8-yl, 1-Nonin-9-yl, 1-Decin-10-yl oder 1-Tetracosin-24-yl.

$C_7$-$C_{18}$-Aralkyl ist zum Beispiel 2-Benzyl-2-propyl, β-Phenyl-ethyl, α,α-Dimethylbenzyl, ω-Phenyl-butyl, ω-Phenyl-octyl, ω-Phenyl-dodecyl oder 3-Methyl-5-(1',1',3',3'-tetramethyl)butyl-benzyl. $C_7$-$C_{24}$-Aralkyl kann darüberhinaus beispielsweise auch 2,4,6-Tri-tert.-butyl-benzyl oder 1-(3,5-Dibenzyl-phenyl)-3-methyl-2-propyl bedeuten.

Sind $R_1$ und $R_1$' substituiert, so handelt es sich bei den Substituenten gegebenenfalls um unter den Reaktionsbedingungen inerte Substituenten. Beispiele von inerten Substituenten sind Halogenatome, wie beispielsweise Fluor oder Chlor, Ethergruppen wie -O-$R_2$ oder -(O-$C_1$-$C_6$)$_n$-O-$C_1$-$C_{18}$-Alkyl, beispielsweise Methoxy, Ethoxy, Butoxy, Octadecyloxy, 3-Oxa-hept-1-yloxy oder Monomethoxy- und Monoethoxy-Polyethylenglykol- oder -Polypropylenglykol-Reste, Aminogruppen wie -$NR_2R_3$, beispielsweise Dimethylamino, Methylethylamino, Diethylamino, Dibutylamino, Butyldodecylamino, Dioctadecylamino oder Methyl-(3-aza-hept-1-yl)-amino, Thioethergruppen wie -S-$R_2$ ($R_2 \neq H$), beispielsweise Methyl-thio, Ethylthio, Butylthio oder Octadecylylthio, Cyano, Nitro oder α.β-ungesättigte Keton-Reste. Bekannte Pyrokohlensäurediester der Formel (I) mit inerte Substituenten tragenden Resten $R_1$ und $R_1$' sind beispielsweise Di-(1,1,1,3,3,3-Hexafluor-2-propyl)-dicarbonat oder Di-[1-Methyl-3-(2,6,6-trimethyl-3-oxo-1-cyclohexen-1-yl)-2-propenyl]-dicarbonat.

Heterozyklische aromatische Amine sind zum Beispiel Pyridin, α-, β- oder γ-Picolin, 2,4-, 2,6-, 3,4- oder 3,5-Lutidin, Collidin oder Chinolin.

Unpolare inerte Lösemittel sind solche, welche eine Dielektrizitätskonstante $\varepsilon \leq 10$ haben, mit Wasser nicht mischbar sind und unter den Verfahrensbedingungen weder mit dem Estercarbonat der Formel (II) noch mit dem Sulfochlorid der Formel (IV) reagieren, zum Beispiel aromatische Kohlenwasserstoffe, wie beispielsweise Benzol, Toluol, Xylol, Mesitylen oder Ethylbenzol, aliphatische Kohlenwasserstoffe, wie beispielsweise Pentan, Hexan, Cyclohexan, Heptan, Octan, Decan oder Dekalin, nicht-zyklische Ether, wie beispielsweise Diethylether, Diisopropylether oder Diisobutylether, oder Gemische davon, wie beispielsweise Siedegrenzebenzine oder ®Shell-Sol Produkte.

Bevorzugte Sulfochloride der Formel (IV) sind Benzolsulfochlorid und p-Toluolsulfochlorid. Besonders bevorzugtes Sulfochlorid der Formel (IV) ist p-Toluolsulfochlorid.
Bevorzugte Katalysatorkatione sind solche, worin $R_2$ bis $R_5$ unabhängig voneinander Methyl, Ethyl, Butyl, Benzyl, Octyl, Dodecyl oder Octadecyl sind, insbesondere solche, worin die Summe der in den Gruppen $R_2$ bis $R_5$ enthaltenen Kohlenstoffatome eine Zahl von 10 bis 24 ist.
Besonders bevorzugte Katalysatorkatione sind solche, worin $R_2$ bis $R_5$ Butyl, oder $R_2$ und $R_3$ Methyl, $R_4$ Methyl oder Ethyl, und $R_5$ Benzyl, Dodecyl oder Octadecyl sind.
Nicht nukleophile Katalysatoranione $X^-$ sind beispielsweise $Cl^-$, $Br^-$, $F^-$, $J^-$, $NO_3^-$, $ClO_4^-$, $HSO_4^-$, $PF_6^-$, $B(C_6H_5)_4^-$ oder $BF_4^-$.
Bevorzugte Katalysatoranione sind Bromid und Chlorid, insbesondere Chlorid.
Besonders bevorzugter Katalysator der Formel (V) ist Benzyltrimethylammoniumchlorid.
Bevorzugtes heterozyklisches aromatisches Amin ist Pyridin.
Bevorzugte Lösemittel sind aromatische Kohlenwasserstoffe.
Besonders bevorzugte Lösemittel sind Toluol und Xylol.
Die bevorzugte Menge Sulfochlorid der Formel (IV) beträgt zirka 45 Mol%, bezogen auf (II).
Die bevorzugte Menge Katalysator der Formel (V) beträgt 1,0-1,5 Mol%, bezogen auf (II).
Die bevorzugte Menge heterozyklisches aromatisches Amin beträgt 1-3 Mol%, bezogen auf (II). Die besonders bevorzugte Menge heterozyklisches aromatisches Amin beträgt zirka 3 Mol%, bezogen auf (II).
Die Menge Lösemittel ist nicht kritisch. Bevorzugt wird gerade so viel Lösemittel verwendet, dass das Reaktionsgemisch während der ganzen Reaktion gut rührbar bleibt; je nach herzustellendem Pyrokohlensäurediester kann diese Menge verschieden sein.
Die bevorzugte Reaktionstemperatur beträgt zwischen 0°C und +20°C.
Die besonders bevorzugte Reaktionstemperatur beträgt zwischen 0°C und +10°C.

Die Reaktionszeit hängt von den Mengen Katalysator und heterozyklisches aromatisches Amin sowie von der Temperatur ab. Üblicherweise ist die Umsetzung nach ½ bis 100 Stunden, bevorzugt nach ½ bis 10 Stunden beendet.

Alle benötigten Chemikalien sind bekannt. Sie sind kommerziell erhältlich oder können nach bekannten Methoden hergestellt werden.

Das Verfahren kann in einfachster Weise durchgeführt werden, indem das Lösemittel und alle Edukte (II) bis (V) gleichzeitig oder nacheinander in beliebiger Reihenfolge bei der Reaktionstemperatur ins Reaktionsgefäss eingeführt werden, wobei zweckmässig mindestens ein Teil des Lösemittels vorgelegt, und das Sulfochlorid zuletzt zugegeben werden.

Bevorzugt wird das Estercarbonat der Formel (II) *in situ* nach üblichen Verfahren hergestellt, beispielsweise wie in den obengenannten Referenzen angegeben. Dabei wird ein Alkoholat der Formel (V')

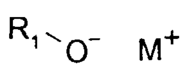 [(V')]

in Lösemittel vorgelegt, was beispielsweise auch durch Umsetzung eines Alkoholes $R_1OH$ mit einem Alkalimetall M oder einem Alkalimetallhydrid MH im vorgenannten Lösemittel unter inerter Atmosphäre geschehen kann. Danach wird Kohlenstoffdioxid, gegebenenfalls unter Druck, eingeleitet, bis sich das Estercarbonat der Formel (II) gebildet hat. Es kann sich als zweckmässig erweisen, vom Temperaturbereich zur Herstellung des Pyrokohlensäurediesters abzuweichen, insbesondere eignen sich zur Beschleunigung und Vervollständigung der Alkoholatbildung aus Alkohol und Metall höhere Temperaturen, zum Beispiel Rückfluss, und zur Mässigung der Alkoholatbildung aus Alkohol und Metallhydrid tiefere Temperaturen, zum Beispiel -15°C.

Danach werden bei der spezifizierten Reaktionstemperatur von zwischen -10°C und +25°C zur Lösung oder Suspension des Estercarbonates der Formel (II) unter Rührung der Katalysator der Formel (V), das heterozyklische aromatische Amin und das Sulfochlorid der Formel (IV) in den zuvor angegebenen Mengen zugegeben. Die Reaktion kann bei Bedarf gaschromatographisch verfolgt werden.

Das Reaktionsgemisch kann dann ebenfalls in üblicher Weise aufarbeitet werden, zum Beispiel durch Waschen mit verdünnter wässriger Säure und/oder mit Wasser und Eindampfen der abgetrennten organischen Phase, wobei letztere mit üblichen, bekannten Mitteln wie Trocknungsmitteln, beispielsweise wasserfreies Natrium- oder Magnesiumsulfat, oder Adsorbentien, beispielsweise Aktivkohle oder Bleicherden, vor dem Eindampfen behandelt werden

kann.

Die Pyrokohlensäurediester der Formel (I) werden in hoher Ausbeute und hoher Reinheit erhalten. Falls erwünscht, können sie zusätzlich noch destilliert werden, wobei diese Destillation infolge der hohen Reinheit des Rohproduktes besonders schonend und rasch durchgeführt werden kann.

Dank der durch die Erfindung erreichten Verbesserung der Reaktivität ist es möglich, Sulfochloridmengen bis nahe an der stöchiometrisch bedingten Grenze von 50 Mol% zu verwenden, ohne Verunreinigung des Produktes durch unreagiertes Sulfochlorid und ohne wesentliche Verlängerung der Reaktionszeit. Bevorzugt ist eine Sulfochloridmenge von zirka 45 Mol%, bezogen auf 100 Mol% Estercarbonat der Formel (II).

Mit Gemischen von Estercarbonaten der Formel (II) können asymmetrische Pyrokohlensäurediester der Formel (I) erhalten werden, worin $R_1$ und $R_1$' verschieden sind. In diesem Fall wird zweckmässig ein Molverhältnis von 1:1 verwendet, wobei bevorzugt das eine Estercarbonat zuerst und das andere Estercarbonat erst nach dem Sulfochlorid zugegeben werden. Je nach Verwendungszweck können solche Pyrokohlensäurediester als Homologengemische verwendet werden, oder die asymmetrische Verbindung kann isoliert werden, zum Beispiel durch fraktionierte Destillation.

Bekannt sind die Pyrokohlensäurediester der Formeln (VI),

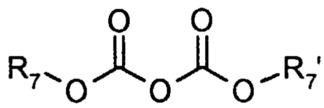 (VI)

worin sowohl $R_7$ als auch $R_7$' unsubstituiertes oder mit einem oder mehreren unter den Reaktionsbedingungen inerten Substituenten substituiertes, in α-Stellung unverzweigtes $C_1$-$C_{24}$-Alkyl darstellen, und (VII),

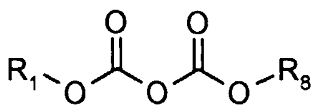 (VII)

worin $R_1$ die zuvor gegebene Definition hat und $R_8$ unsubstituiertes oder mit einem oder mehreren unter den Reaktionsbedingungen inerten Substituenten substituiertes, verzweigtes oder unverzweigtes $C_3$-$C_{24}$-Alkenyl, $C_3$-$C_{24}$-Alkinyl, $C_4$-$C_{12}$-Cycloalkyl, $C_4$-$C_{12}$-Cycloalkenyl oder $C_7$-$C_{24}$-Aralkyl,
oder in α-Stellung verzweigtes $C_3$-$C_{24}$-Alkyl darstellt,
wobei $R_1$ und $R_8$ in Formel (VII) derart kombiniert sind, wie in Tabelle 1 angegeben:

**Tabelle 1** : bekannte Verbindungen der Formel (VII)

| Verbindung | C.A. Reg.-Nr. | $R_1$ | $R_8$ |
|---|---|---|---|
| VII a | 4525-34-2 | 2-Propinyl | 2-Propinyl |
| VII b | 5944-45-6 | Methyl | Allyl |
| VII c | 5944-46-7 | Allyl | Benzyl |
| VII d | 5944-47-8 | Ethyl | Benzyl |
| VII e | 24424-99-5 | tert.-Butyl | tert.-Butyl |
| VII f | 24425-00-1 | Isopropyl | Isopropyl |
| VII g | 31139-36-3 | Benzyl | Benzyl |
| VII h | 32813-64-2 | 2-Benzyl-2-propyl | 2-Benzyl-2-propyl |
| VII i | 55130-19-3 | Isopropyl | tert.-Butyl |
| VII j | 61114-48-5 | Cyclohexyl | Cyclohexyl |
| VII k | 61114-49-6 | 2-Butyl | 2-Butyl |
| VII l | 65815-74-9 | 1-Adamantyl | 1-Adamantyl |
| VII m | 68835-89-2 | 2-Methyl-2-butyl | 2-Methyl-2-butyl |
| VII n | 115491-93-5 | Allyl | Allyl |
| VII o | 116055-32-4 | Ethyl | 3,4-Dichlorbenzyl |
| VII p | 116977-36-7 | Ethyl | Allyl |
| VII q | 125372-96-5 | 1,1,1,3,3,3-Hexafluor-2-propyl | 1,1,1,3,3,3-Hexafluor-2-propyl |
| VII r | 133389-94-3 | 3-Butenyl | 3-Butenyl |
| VII s | 146919-25-7 | tert.-Butyl | 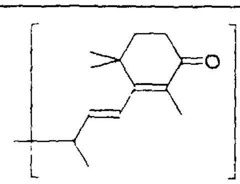 |
| VII t | 149364-65-8 | Cyclopentyl | Cyclopentyl |
| VII u | 160788-62-5 | Allyl | 1-Methylethenyl |
| VII v | – | Dimethyl-1-benzyl-ethyl | Dimethyl-1-benzyl-ethyl |
| VII w | – | Methyl | tert-Butyl |

| | | | |
|---|---|---|---|
| VII x | = | Isobutyl | tert-Butyl |
| VII y | = | Ethyl | tert-Butyl |
| VII z | = | Undecafluoro-2-pentyl | Undecafluoro-2-pentyl |
| VII ø | = | p-tert-Butyl-benzyl | p-tert-Butyl-benzyl |
| VII ð | = | Ethyl | 3,3,7-Trimethyl-cyclohepta-1,4,6-trienyl |

Die übrigen nach dem erfindungsgemässen Verfahren herstellbaren Pyrokohlensäurediester der Formel (I) sind noch neu.

Die Erfindung betrifft daher Pyrokohlensäurediester der Formel (VIII),

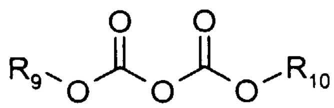 (VIII)

worin

$R_9$ unsubstituiertes oder mit einem oder mehreren unter den Reaktionsbedingungen inerten Substituenten substituiertes verzweigtes oder unverzweigtes $C_1$-$C_{24}$-Alkyl, $C_3$-$C_{24}$-Alkenyl, $C_3$-$C_{24}$-Alkinyl, $C_4$-$C_{12}$-Cycloalkyl, $C_4$-$C_{12}$-Cycloalkenyl oder $C_7$-$C_{24}$-Aralkyl bedeutet, und
$R_{10}$ unabhängig von $R_9$ unsubstituiertes oder mit einem oder mehreren unter den Reaktionsbedingungen inerten Substituenten substituiertes, verzweigtes oder unverzweigtes $C_3$-$C_{24}$-Alkenyl, $C_3$-$C_{24}$-Alkinyl, $C_4$-$C_{12}$-Cycloalkyl, $C_4$-$C_{12}$-Cycloalkenyl oder $C_7$-$C_{24}$-Aralkyl, oder in $\alpha$-Stellung verzweigtes $C_3$-$C_{24}$-Alkyl bedeutet,
mit der Massgabe, dass
wenn $R_9$ gleich Methyl ist, $R_{10}$ nicht Allyl,
wenn $R_9$ gleich Ethyl ist, $R_{10}$ weder Allyl noch Benzyl noch 3,4-Dichlorbenzyl,
wenn $R_9$ gleich 2-Propinyl ist, $R_{10}$ nicht 2-Propinyl,
wenn ein Rest $R_9$ oder $R_{10}$ gleich Allyl ist,
der andere Rest $R_{10}$ oder $R_9$ weder Allyl noch 1-Methylethenyl noch Benzyl,
wenn ein Rest $R_9$ oder $R_{10}$ gleich Isopropyl ist,
der andere Rest $R_{10}$ oder $R_9$ weder Isopropyl noch tert.-Butyl,
wenn $R_9$ gleich 1,1,1,3,3,3-Hexafluor-2-propyl ist,
$R_{10}$ nicht 1,1,1,3,3,3-Hexafluor-2-propyl,
wenn ein Rest $R_9$ oder $R_{10}$ gleich tert.-Butyl ist, der andere Rest $R_{10}$ oder $R_9$ weder tert.-Butyl noch 1-Methyl-3-(2,6,6-trimethyl-3-oxo-1-cyclohexen-1-yl)-2-propenyl,
wenn $R_9$ gleich 2-Butyl ist, $R_{10}$ nicht 2-Butyl,
wenn $R_9$ gleich 3-Butenyl ist, $R_{10}$ nicht 3-Butenyl,
wenn $R_9$ Cyclopentyl ist, $R_{10}$ nicht Cyclopentyl,
wenn $R_9$ gleich Cyclohexyl ist, $R_{10}$ nicht Cyclohexyl,
wenn $R_9$ gleich 2-Methyl-2-butyl ist, $R_{10}$ nicht 2-Methyl-2-butyl,
wenn $R_9$ gleich Benzyl ist, $R_{10}$ nicht Benzyl,
wenn $R_9$ gleich 2-Benzyl-2-propyl ist, $R_{10}$ nicht 2-Benzyl-2-propyl,
wenn $R_9$ gleich 1-Adamantyl ist, $R_{10}$ nicht 1-Adamantyl,
wenn $R_9$ gleich 1-Dimethyl-1-benzyl-ethyl ist, $R_{10}$ nicht 1-Dimethyl-1-benzyl-ethyl,
wenn $R_9$ gleich Methyl ist, $R_{10}$ nicht tert-Butyl,
wenn $R_9$ gleich Isobutyl ist, $R_{10}$ nicht tert-Butyl,
wenn $R_9$ gleich Ethyl ist, $R_{10}$ nicht tert-Butyl,
wenn $R_9$ gleich Undecafluoro-2-pentyl ist, $R_{10}$ nicht Undecafluoro-2-pentyl,

wenn $R_9$ gleich p-tert-Butyl-benzyl ist, $R_{10}$ nicht p-tert-Butyl-benzyl,
wenn $R_9$ gleich Ethyl ist, $R_{10}$ nicht 3,3,7-Trimethyl-cyclohepta-1,4,6-trienyl,
wenn $R_9$ gleich Methyl ist, $R_{10}$ nicht Methyl,
wenn $R_9$ gleich Ethyl ist, $R_{10}$ nicht Ethyl,
wenn $R_9$ gleich n-Propyl ist, $R_{10}$ nicht n-Propyl,
wenn $R_9$ gleich Isobutyl ist, $R_{10}$ nicht Isobutyl,
wenn $R_9$ gleich Allyl ist, $R_{10}$ nicht Allyl,
wenn $R_9$ gleich n-Butyl ist, $R_{10}$ nicht n-Butyl,
wenn $R_9$ gleich tert-Amyl ist, $R_{10}$ nicht tert-Amyl,
wenn $R_9$ gleich 2-Ethyl-hexyl ist, $R_{10}$ nicht 2-Ethyl-hexyl,
wenn $R_9$ gleich 4-tert-Butyl-cyclohexyl ist, $R_{10}$ nicht 4-tert-Butyl-cyclohexyl,
wenn $R_9$ gleich Ethyl ist, $R_{10}$ nicht 2-Methyl-2-butyl,
wenn $R_9$ gleich Allyl ist, $R_{10}$ nicht tert-Butyl,
wenn $R_9$ gleich Methyl ist, $R_{10}$ nicht Isopropyl,
wenn $R_9$ gleich Ethyl ist, $R_{10}$ nicht Isopropyl, und
wenn $R_9$ gleich Ethyl ist, $R_{10}$ nicht 2-Butyl,
sind.

Beim Pyrokohlensäurediester der Formel (VIII) handelt es sich zum Beispiel um Di-3-ethyl-3-pentyl-dicarbonat, Di-2-methyl-2-hexyl-dicarbonat, Di-2-benzyl-2-pronyl-dicarbonat, Di-9-methyl-9-octadecyl-dicarbonat, Di-2-methyl-2-decyl-dicarbonat, Di-2-methyl-2-nonadecyl-dicarbonat oder Di-5-nonyl-dicarbonat.

Bevorzugt sind die Pyrokohlensäurediester der Formel (I), worin $R_1$ und $R_1'$ mit einem sekundären oder tertiären Kohlenstoffatom am Sauerstoffatom der Dicarbonatgruppe gebunden sind, sowie solche, worin $R_1$ und $R_1'$ in β-Stellung ungesättigt sind, insbesondere Di-tert.-butyl-dicarbonat, Di-tert.-pentyl-dicarbonat, Di-5-nonyl-dicarbonat, Di-allyl-dicarbonat, Di-(2-methyl-3-butin-2-yl)-dicarbonat oder Di-(2-methyl-3-buten-2-yl)-dicarbonat.
Besonders bevorzugte Pyrokohlensäurediester der Formel (I) sind Di-tert.-pentyl-dicarbonat, Di-5-nonyl-dicarbonat, Di-allyl-dicarbonat, Di-(2-methyl-3-buten-2-yl)-dicarbonat oder Di-(2-methyl-3-butin-2-yl)-dicarbonat.
Die Pyrokohlensäurediester der Formel (VIII) können für jeden beliebigen bekannten Zweck verwendet werden; bevorzugt können sie zur Herstellung löslicher Pigmentvorläufer verwendet werden, beispielsweise solcher, welche in EP 648 770 und EP 648 817 beschrieben werden.
Bevorzugte Pigmentvorläufer, zu deren Herstellung Pyrokohlensäurediester der Formel (VIII) verwendet werden können, sind solche der Formel (IX),

$$A(B)_x \qquad (IX)$$

worin

x eine ganze Zahl von 1 bis 4,
A der Rest eines Chromophores der Chinacridon-, Anthrachinon-, Indanthron-, Perylen-, Indigo-, Chinophthalon-, Isoindolinon-, Isoindolin-, Dioxazin-, Azo-, Phthalocyanin- oder Diketopyrrolopyrrol-Reihe, welches x mit mindestens einer Carbonylgruppe konjugierten oder benachbarten N-Atome enthält, und
B eine mit einem dieser N-Atome verbundene Gruppe der Formel (X)

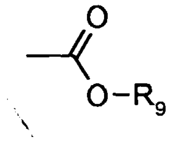 (X)

worin $R_9$ die oben angegebene Bedeutung hat, sind.

Die Chromophorreste und Substitutionsmuster solcher Pigmentvorläufer, sowie einzelne Pigmentvorläufer selbst, sind beispielsweise aus EP 654 711 bekannt.
Die folgenden Beispiele veranschaulichen die Erfindung:

Beispiele 1-10 (allgemeine Vorschrift):

0,1 mol Alkalimetall-alkoxid werden in 150 ml Toluol suspendiert (oder gelöst); dann wird bei 0-10°C 1,2 mol $CO_2$ eingeleitet. Zur gerührten Suspension (Lösung) werden 228 mg Benzyltrimethylammoniumchlorid und 240 mg Pyridin, nach 15 Min die unten angegebene Menge Toluol-4-sulfochlorid gegeben. Die entstehende Suspension wird über Nacht weitergerührt. Die entstandene dicke Suspension wird nach Zugabe von 25 ml 5%ige wässerige $H_2SO_4$ 30 Min gerührt, dann wird die organische Phase abgetrennt, dreimal mit je 150 ml Wasser gewaschen, über $MgSO_4$ getrocknet und mit Aktivkohle behandelt. Anschliesslich wird die farblose Lösung unter Vakuum eingeengt. Man erhält die Pyrokohlensäurediester in guter Reinheit mit den in Tabelle 2 angegebenen Ausbeuten und chromatographischen Retentionswerten.

Tabelle 2 :

| Beispiele 1-10 | | | | |
|---|---|---|---|---|
| **Beispiel Nr.** | **$R_1$ = $R_1$'** | **mol (IV)** | **Ausbeute** [ bezogen auf (IV) ] | **$R_f$** (Kieselgel, Hexan : $CH_2Cl_2$ 1:1) |
| **1** | tert.-Butyl | 0,40 | 85% | 0,55 |
| **2** | 2-Methyl-2-butyl | 0,43 | 81% | 0,61 |
| **3** | 3-Ethyl-3-pentyl | 0,42 | 58% | 0,63 |
| **4** | 2-Methyl-2-hexyl | 0,42 | 37% | 0,67 |
| **5** | 2-Benzyl-2-propyl | 0,40 | 29% | 0,50 |
| **6** | Isopropyl | 0,40 | 55% | 0,62 |
| **7** | 9-Methyl-9-octadecyl | 0,43 | 93% | 0,45 |
| **8** | 2-Methyl-2-decyl | 0,42 | 65% | 0,32 |
| **9** | 2-Methyl-2-nonadecyl | 0,43 | 98% | 0,19 |
| **10** | 5-Nonyl | 0,40 | 75% | 0,76 |

Die $^1$H-Kernresonanzspektren ($^1$H-NMR) der Produkte stimmen mit den bekannten Spektren überein oder sind in gutem Einklang mit der Erwartung.

Beispiel 11:

Beispiel 1 wird wiederholt, jedoch nimmt man anstatt 0,40 mol Toluol-4-sulfochlorid 0,45 mol Toluol-4-sulfochlorid. Man erhält Di-tert.-butyl-dicarbonat in vergleichbarer Qualität.

Beispiel 12:

Beispiel 1 wird wiederholt, jedoch nimmt man anstatt 0,40 mol Toluol-4-sulfochlorid 0,48 mol Toluol-4-sulfochlorid. Man erhält Di-tert.-butyl-dicarbonat in vergleichbarer Qualität.

Beispiel 13:

Einer auf 3°C gekühlten Lösung von 84,1 g (1 mol) 2-Methyl-3-butin-2-ol in 1,75 l Toluol werden unter Schutzgasatmosphäre 40 g (1 mol) 60%iges Natriumhydrid portionenweise so zugegeben, dass die Temperatur 10°C nicht übersteigt. Nach Zugabe von 250 ml Toluol wird das Gemisch über Nacht bei 18°C gerührt. Nach Abkühlung der braunen Lösung auf 5°C werden 101,7 g (2,3 mol) $CO_2$ bei 5-10°C eingeleitet. Zum auf 18°C erwärmten Reaktionsgemisch werden nacheinander 3,2 g (0,014 mol) Benzyltrimethylammoniumchlorid, 2,4 g (0,03 mol) Pyridin und 82,8 g (0,43 mol) Toluol-4-sulfochlorid gegeben. Die entstehende Suspension wird drei Tage bei Raumtemperatur weitergerührt. Bei 5°C werden dann 260 ml 5%ige wässerige $H_2SO_4$ so zugetropft, dass die Temperatur 10°C nicht übersteigt. Die organische Phase wird abgetrennt, fünfmal mit je 400 ml Wasser gewaschen, über $Na_2SO_4$ getrocknet und im Vakuum eingeengt. Das Rohprodukt (94,8 g) wird mit Hexan behandelt, wobei man 70 g (59% d.Th., berechnet auf das eingesetzte Toluol-4-sulfochlorid) reines weisses Di-(2-methyl-3-butin-2-yl)-dicarbonat vom Schmelzpunkt 102,8°C bekommt. Die Elementaranalyse und das $^1$H-NMR-Spektrum sind in gutem Einklang mit der Erwartung.

Beispiel 14:

Beispiel 13 wird wiederholt, jedoch ausgehend von 2-Methyl-3-buten-2-ol anstatt 2-Methyl-3-butin-2-ol, und mit 0,50 mol Toluol-4-sulfochlorid anstatt 0,40 mol Toluol-4-sulfochlorid. Man erhält Di-(2-methyl-3-buten-2-yl)-dicarbonat in guter Qualität mit einer Ausbeute von 61% d.Th. (berechnet auf das eingesetzte Toluol-4-sulfochlorid).
Das $^1$H-NMR-Spektrum ist in gutem Einklang mit der Erwartung.

Beispiel 15:

Beispiel 13 wird wiederholt, jedoch ausgehend von 3-Methyl-2-buten-1-ol anstatt 2-Methyl-3-butin-2-ol. Man erhält Di-(3-methyl-2-buten-1-yl)-dicarbonat mit einem $^1$H-NMR-Spektrum in gutem Einklang mit der Erwartung.

Beispiel 16:

6,6 g (0,15 mol) $CO_2$ werden zu einer Suspension von 11,22 g (0,1 mol) Kalium-tert.-butylat in 150 ml Toluol bei 0-10°C eingeleitet. Zur gerührten Suspension werden 228 mg Benzyltrimethylammoniumchlorid, 240 mg Pyridin, und nach 15 Min 7,6 g (0,04) mol Toluol-4-sulfochlorid gegeben. Die entstehende Suspension wird über Nacht weitergerührt. Die entstandene dicke Suspension wird nach Zugabe von 25 ml 5%ige wässerige $H_2SO_4$ 30 Min gerührt, dann wird die organische Phase abgetrennt, dreimal mit je 150 ml Wasser gewaschen, über $MgSO_4$ getrocknet und mit Aktivkohle behandelt. Anschliesslich wird die farblose Lösung unter Vakuum eingeengt. Der farblose Rückstand wird bei 55°C/$10^{-2}$ mbar destilliert. Die destillierte Fraktion ergibt 6,37 g reines Di-tert.-butyl-dicarbonat; Ausbeute 73% d. Th. (berechnet auf das eingesetzte Toluol-4-sulfochlorid).

Beispiel 17:

6,6 g (0,15 mol) $CO_2$ werden zu einer Suspension von 11,22 g (0,1 mol) Kalium-tert.-butylat in 150 ml Toluol bei 0-10°C eingeleitet. Zur gerührten Suspension werden 228 mg Benzyltrimethylammoniumchlorid, 240 mg Pyridin, und nach 15 Min 8,58 g (0,045) mol Toluol-4-sulfochlorid gegeben. Die entstehende Suspension wird über Nacht weitergerührt. Die entstandene dicke Suspension wird nach Zugabe von 25 ml 5%ige wässerige $H_2SO_4$ 30 Min gerührt, dann wird die organische Phase abgetrennt, dreimal mit je 150 ml Wasser gewaschen, über $MgSO_4$ getrocknet und mit Aktivkohle behandelt. Anschliesslich wird die farblose Lösung unter Vakuum eingeengt. Der farblose Rückstand wird bei 55°C/$10^{-2}$ mbar destilliert.
Die destillierte Fraktion ergibt 6,37 g reines Di-tert.-butyl-dicarbonat; Ausbeute 80% d.Th. (berechnet auf das eingesetzte Toluol-4-sulfochlorid).

Beispiel 18:

Zu einer gerührten Suspension von 14,01 g (0,1 mol) Natrium-tert.-butylcarbonat in 150 ml Toluol werden bei 20°C 228 mg Benzyltrimethylammoniumchlorid, 240 mg Pyridin und nach 15 Min 7,6 g (0,04 mol) Toluol-4-sulfochlorid gegeben. Die entstehende Suspension wird 24 Std bei 40 °C weitergerührt. Die entstandene dicke Suspension wird nach Zugabe von 25 ml 5%ige wässerige $H_2SO_4$ 30 Min gerührt, dann wird die organische Phase abgetrennt, dreimal mit je 150 ml Wasser gewaschen, über $MgSO_4$ getrocknet und mit Aktivkohle behandelt. Anschliesslich wird die farblose Lösung unter Vakuum eingeengt. Der farblose Rückstand wird bei 55°C/$10^{-2}$ mbar destilliert. Die destillierte Fraktion ergibt 7,2 g reines Di-tert.-butyl-dicarbonat; Ausbeute 82,5 % d.Th. (berechnet auf das eingesetzte Toluol-4-sulfochlorid).

Vergleichsbeispiel 1:

(zu Beispiel 18 / Reaktionsbedingungen nach CS 260076, Beispiel 2): Zu einer gerührten Suspension von 14,01 g (0,1 mol) Natrium-tert.-butylcarbonat in 150 ml Toluol werden bei 20 °C 228 mg Benzyltrimethylammoniumchlorid, 10 ml Pyridin und nach 15 Min 7,6 g (0,04 mol) Toluol-4-sulfochlorid gegeben. Die entstehende Suspension wird für 1,5 Std bei 50°C weitergerührt. Die entstandene dicke Suspension wird nach Zugabe von 25 ml 5%ige wässerige $H_2SO_4$ 30 Min gerührt, dann wird die organische Phase abgetrennt, dreimal mit je 150 ml Wasser gewaschen, über $MgSO_4$ getrocknet und mit Aktivkohle behandelt. Anschliesslich wird die braune Lösung unter Vakuum eingeengt. Der dunkelbraune Rückstand wird bei 55°C/$10^{-2}$ mbar destilliert. Die destillierte Fraktion ergibt 5,8 g eines Gemisches, das aus 93,7% Di-tert.-butyl-dicarbonat und 6,3% unreagiertem Toluol-4-sulfochlorid besteht. Die Ausbeute Di-tert.-butyl-dicarbonat im Gemisch beträgt 62,7% d.Th. (berechnet auf das eingesetzte Toluol-4-sulfochlorid).

## Patentansprüche

1. Pyrokohlensäurediester der Formel (VIII),

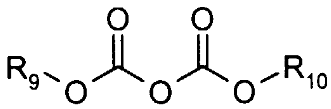

(VIII)

worin $R_9$ unsubstituiertes oder mit einem oder mehreren unter den Reaktionsbedingungen inerten Substituenten substituiertes verzweigtes oder unverzweigtes $C_1$-$C_{24}$-Alkyl, $C_3$-$C_{24}$-Alkenyl, $C_3$-$C_{24}$-Alkinyl, $C_4$-$C_{12}$-Cycloalkyl, $C_4$-$C_{12}$-Cycloalkenyl oder $C_7$-$C_{24}$-Aralkyl bedeutet, und
$R_{10}$ unabhängig von $R_9$ unsubstituiertes oder mit einem oder mehreren unter den Reaktionsbedingungen inerten Substituenten substituiertes, verzweigtes oder unverzweigtes $C_3$-$C_{24}$-Alkenyl, $C_3$-$C_{24}$-Alkinyl, $C_4$-$C_{12}$-Cycloalkyl, $C_4$-$C_{12}$-Cycloalkenyl oder $C_7$-$C_{24}$-Aralkyl, oder in $\alpha$-Stellung verzweigtes $C_3$-$C_{24}$-Alkyl bedeutet, mit der Massgabe, dass

wenn $R_9$ gleich Methyl ist, $R_{10}$ nicht Allyl,
wenn $R_9$ gleich Ethyl ist, $R_{10}$ weder Allyl noch Benzyl noch 3,4-Dichlorbenzyl,
wenn $R_9$ gleich 2-Propinyl ist, $R_{10}$ nicht 2-Propinyl,
wenn ein Rest $R_9$ oder $R_{10}$ gleich Allyl ist, der andere Rest $R_{10}$ oder $R_9$ weder Allyl noch 1-Methylethenyl noch Benzyl,
wenn ein Rest $R_9$ oder $R_{10}$ gleich Isopropyl ist, der andere Rest $R_{10}$ oder $R_9$ weder Isopropyl noch tert.-Butyl,
wenn $R_9$ gleich 1,1,1,3,3,3-Hexafluor-2-propyl ist, $R_{10}$ nicht 1,1,1,3,3,3-Hexafluor-2-propyl,
wenn ein Rest $R_9$ oder $R_{10}$ gleich tert.-Butyl ist, der andere Rest $R_{10}$ oder $R_9$ weder tert.-Butyl noch 1-Methyl-3-(2,6,6-trimethyl-3-oxo-1-cyclohexen-1-yl)-2-propenyl,
wenn $R_9$ gleich 2-Butyl ist, $R_{10}$ nicht 2-Butyl,
wenn $R_9$ gleich 3-Butenyl ist, $R_{10}$ nicht 3-Butenyl,
wenn $R_9$ Cyclopentyl ist, $R_{10}$ nicht Cyclopentyl,
wenn $R_9$ gleich Cyclohexyl ist, $R_{10}$ nicht Cyclohexyl,
wenn $R_9$ gleich 2-Methyl-2-butyl ist, $R_{10}$ nicht 2-Methyl-2-butyl,

wenn $R_9$ gleich Benzyl ist, $R_{10}$ nicht Benzyl,
wenn $R_9$ gleich 2-Benzyl-2-propyl ist, $R_{10}$ nicht 2-Benzyl-2-propyl,
wenn $R_9$ gleich 1-Adamantyl ist, $R_{10}$ nicht 1-Adamantyl,
wenn, $R_9$ gleich 1-Dimethyl-1-benzyl-ethyl ist, $R_{10}$ nicht 1-Dimethyl-1-benzyl-ethyl
wenn $R_9$ gleich Methyl ist, $R_{10}$ nicht tert-Butyl,
wenn $R_9$ gleich Isobutyl ist, $R_{10}$ nicht tert-Butyl,
wenn $R_9$ gleich Ethyl ist, $R_{10}$ nicht tert-Butyl,
wenn $R_9$ gleich Undecafluoro-2-pentyl ist, $R_{10}$ nicht Undecafluoro-2-pentyl,
wenn $R_9$ gleich p-tert-Butyl-benzyl ist, $R_{10}$ nicht p-tert-Butyl-benzyl,
wenn $R_9$ gleich Ethyl ist, $R_{10}$ nicht 3,3,7-Trimethyl-cyclohepta-1,4,6-trienyl,
wenn $R_9$ gleich Methyl ist, $R_{10}$ nicht Methyl,
wenn $R_9$ gleich Ethyl ist, $R_{10}$ nicht Ethyl,
wenn $R_9$ gleich n-Propyl ist, $R_{10}$ nicht n-Propyl,
wenn $R_9$ gleich Isobutyl ist, $R_{10}$ nicht Isobutyl,
wenn $R_9$ gleich Allyl ist, $R_{10}$ nicht Allyl,
wenn $R_9$ gleich n-Butyl ist, $R_{10}$ nicht n-Butyl,
wenn $R_9$ gleich tert-Amyl ist, $R_{10}$ nicht tert-Amyl,
wenn $R_9$ gleich 2-Ethyl-hexyl ist, $R_{10}$ nicht 2-Ethyl-hexyl,
wenn $R_9$ gleich 4-tert-Butyl-cyclohexyl ist, $R_{10}$ nicht 4-tert-Butyl-cyclohexyl,
wenn $R_9$ gleich Ethyl ist, $R_{10}$ nicht 2-Methyl-2-butyl,
wenn $R_9$ gleich Allyl ist, $R_{10}$ nicht tert-Butyl,
wenn $R_9$ gleich Methyl ist, $R_{10}$ nicht Isopropyl,
wenn $R_9$ gleich Ethyl ist, $R_{10}$ nicht Isopropyl, und
wenn $R_9$ gleich Ethyl ist, $R_{10}$ nicht 2-Butyl,

sind.

**2.** Verwendung eines Pyrokohlensäurediesters nach Anspruch 1 zur Herstellung löslicher Pigmentvorläufer.

**3.** Verwendung nach Anspruch 2, worin der Pigmentvorläufer eine Verbindung der Formel (IX) ist,

$$A(B)_x \qquad (IX)$$

worin x eine ganze Zahl von 1 bis 4,

A der Rest eines Chromophores der Chinacridon-, Anthrachinon-, Indanthron-, Perylen-, Indigo-, Chinophthalon-, Isoindolinon-, Isoindolin-, Dioxazin-, Azo-, Phthalocyanin- oder Diketopyrrolopyrrol-Reihe, welches x mit mindestens einer Carbonylgruppe konjugierten oder benachbarten N-Atome enthält, und
B eine mit einem dieser N-Atome verbundene Gruppe der Formel (X)

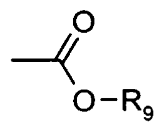 (X)

worin $R_9$ die im Anspruch 1 angegebene Bedeutung hat.

**4.** Pyrokohlensäurediester nach Anspruch 1, wobei es sich dabei um Di-3-ethyl-3-pentyl-dicarbonat, Di-2-methyl-2-hexyl-dicarbonat, Di-2-benzyl-2-propyl-dicarbonat, Di-9-methyl-9-octadecyl-dicarbonat, Di-2-methyl-2-decyl-dicarbonat, Di-2-methyl-2-nonadecyl-dicarbonat, Di-5-nonyl-dicarbonat, Di-(2-methyl-3-buten-2-yl)-dicarbonat oder Di-(2-methyl-3-butin-2-yl)-dicarbonat handelt.

## Claims

**1.** A pyrocarbonic diester of formula (VIII)

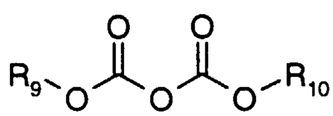 (VIII)

wherein $R_9$ is branched or straight-chain $C_1$-$C_{24}$alkyl, $C_3$-$C_{24}$alkenyl, $C_3$-$C_{24}$alkynyl, $C_4$-$C_{12}$cycloalkyl, $C_4$-$C_{12}$cycloalkenyl or $C_7C_{24}$aralkyl, each of which is unsubstituted or substituted by one or more than one substituent which is inert under the reaction conditions, and
$R_{10}$ is independently of $R_9$ branched or straight-chain $C_3$-$C_{24}$alkenyl, $C_3$-$C_{24}$alkynyl, $C_4$-$C_{12}$cycloalkyl, $C_4$-$C_{12}$cycloalkenyl or $C_7C_{24}$aralkyl, each of which is unsubstituted or substituted by one or more than one substituent which is inert under the reaction conditions, or is $C_3$-$C_{24}$alkyl which is branched in $\alpha$-position,
with the proviso that

if $R_9$ is methyl, $R_{10}$ is not allyl,
if $R_9$ is ethyl, $R_{10}$ is neither allyl nor benzyl nor 3,4-dichlorobenzyl,
if $R_9$ is 2-propynyl, $R_{10}$ is not 2-propynyl,
if either $R_9$ or $R_{10}$ is allyl,
then the remaining $R_{10}$ or $R_9$ is neither allyl nor 1-methylethenyl nor benzyl,
if either $R_9$ or $R_{10}$ is isopropyl,
then the remaining $R_{10}$ or $R_9$ is neigher isopropyl nor tert-butyl,

if $R_9$ is 1,1,1,3,3,3-hexafluoro-2-propyl,
$R_{10}$ is not 1,1,1,3,3,3-hexafluoro-2-propyl,
if either $R_9$ or $R_{10}$ is tert-butyl, then the remaining $R_{10}$ or $R_9$ is neither tert-butyl nor 1 methyl-3-(2,6,6-trimethyl-3-(2,6,6-trimethyl-3-oxo-1-cyclohexen-1-yl)-2-propenyl,
if $R_9$ is butyl, $R_{10}$ is not 2-butyl,
if $R_9$ is 3-butenyl, $R_{10}$ is not-3-butenyl,
if $R_9$ is cyclopentyl, $R_{10}$ is not cyclopentyl,
if $R_9$ is cyclohexyl, $R_{10}$ is not cyclohexyl,
if $R_9$ is 2-methyl-2-butyl, $R_{10}$ is not 2-methyl-2-butyl,
if $R_9$ is benzyl, $R_{10}$ is not benzyl,
if $R_9$ is 2-benzyl-2-propyl, $R_{10}$ is not 2-benzyl-2-propyl,
if $R_9$ is 1-adamantyl, $R_{10}$ is not 1-adamanthyl,
if $R_9$ is 1-dimethyl-1-benzyl ethyl, $R_{10}$ is not 1-dimethyl-1-benzyl ethyl,
if $R_9$ is methyl, $R_{10}$ is not tert-butyl,
if $R_9$ is isobuthyl, $R_{10}$ is not tert-butyl,
if $R_9$ ethyl, $R_{10}$ is not tert-butyl,
if $R_9$ is undecafluoro-2-pentyl, $R_{10}$ is not undecafluoro-2-pentyl,
if $R_9$ is p-tert-butyl-benzyl, $R_{10}$ is not p-tert-butyl-benzyl,
if $R_9$ is ethyl, $R_{10}$ is not 3,3,7-trimethyl-cyclohepta-1,4,6-trienyl,
if $R_9$ is methyl, $R_{10}$ is not methyl,
if $R_9$ is ethyl, $R_{10}$ is not ethyl,
if $R_9$ is n-propyl, $R_{10}$ is not n-propyl,
if $R_9$ is isobuthyl, $R_{10}$ is not isobutyl,
if $R_9$ is allyl, $R_{10}$ is not allyl,
if $R_9$ is n-butyl, $R_{10}$ is not n-butyl,
if $R_9$ tert-amyl, $R_{10}$ is not tert-amyl,
if $R_9$ is 2-ethyl-hexyl, $R_{10}$ is not 2-ethyl-hexyl,
if $R_9$ 4-tert-butyl-cyclohexyl, $R_{10}$ is not 4-tert-butyl-cyclohexyl,
if $R_9$ is ethyl, $R_{10}$ is not 2-methyl-2-butyl,
if $R_9$ is allyl, $R_{10}$ is not tert-butyl,
if $R_9$ is methyl, $R_{10}$ is not isopropyl,
if $R_9$ ethyl, $R_{10}$ is not isopropyl, and
if $R_9$ is ethyl, $R_{10}$ is not 2-butyl.

**2.** The use of a pyrocarbonic diester according to claim 1 for the preparation of a soluble pigment precursor.

**3.** The use according to claim 2, wherein the pigment precursor is a compound of formula (IX)

$$A(B)_x \qquad (IX)$$

wherein x is an integer from 1 to 4,
A is the radical of a chromophore of the quinacridone, anthraquinone, indanthrone, perylene, indigo, quinophthalone, isoindolinone, isoindoline, dioxazine, azo, phthalocyanine or diketopyrrolopyrrole series, which radical A contains x N atoms which are conjugated with, or adjacent to, at least one carbonyl group, and
B is a group of formula (X)

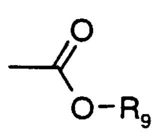
(X)

which is linked to one of these N atoms and wherein $R_9$ has the meaning given in claim 1.

**4.** The pyrocarbonic diester according to claim 1, which is di-3-ethyl-3-pentyl dicarbonate, di-2-methyl-2-hexyl dicarbonate, di-2-benzyl-2-propyl dicarbonate, di-9-methyl-9-octadecyl dicarbonate, di-2-methyl-2-decyl dicarbonate,

di-2-methyl-2-nonadecyl dicarbonate, di-5-nonyl dicarbonate, di(2-methyl-3-buten-2-yl) dicarbonate or di(2-methyl-3-butyn-2-yl) dicarbonate.

## Revendications

1. Diester d'acide pyrocarbonique de formule (VIII),

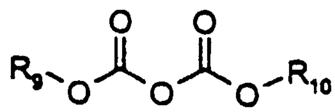
(VIII)

dans laquelle
$R_9$ représente un groupe alkyle en $C_1$ à $C_{24}$, alcényle en $C_3$ à $C_{24}$, alcynyle en $C_3$ à $C_{24}$, cycloalkyle en $C_4$ à $C_{12}$, cycloalcényle en $C_4$ à $C_{12}$ ou aralkyle en $C_7$ à $C_{24}$ non ramifié ou ramifié, non substitué ou substitué avec un ou plusieurs substituants inertes dans les conditions de réaction, et $R_{10}$ représente indépendamment de $R_9$ un groupe alcényle en $C_3$ à $C_{24}$, alcynyle en $C_3$ à $C_{24}$, cycloalkyle en $C_4$ à $C_{12}$, cycloalcényle en $C_4$ à $C_{12}$ ou aralkyle en $C_7$ à $C_{24}$ non ramifié ou ramifié, ou alkyle en $C_3$ à $C_{24}$ ramifié en position a, non substitué ou substitué avec un ou plusieurs substituants inertes dans les conditions de réaction,
avec la condition que

lorsque $R_9$ est un méthyle, $R_{10}$ n'est pas un allyle,
lorsque $R_9$ est un éthyle, $R_{10}$ n'est ni un allyle ni un benzyle, ni un 3,4-Dichlorobenzyle,
lorsque $R_9$ est un 2-Propynyle, $R_{10}$ n'est pas un 2-Propynyle,
lorsqu'un groupe $R_9$ ou $R_{10}$ est un Allyle, l'autre groupe $R_{10}$ ou $R_9$ n'est ni un Allyle ni un 1-Méthyléthényle ni un benzyle,
lorsqu'un groupe $R_9$ ou $R_{10}$ est un Isopropyle, l'autre groupe $R_{10}$ ou $R_9$ n'est ni un Isopropyle ni un tert.-butyle,
lorsque R9 est un 1,1,1,3,3,3-Hexafluoro-2-propyle, $R_{10}$ n'est pas un 1,1,1,3,3,3-Hexafluoro-2-propyle,
lorsqu'un groupe $R_9$ ou $R_{10}$ est un tert.-butyle, l'autre groupe $R_{10}$ ou $R_9$ n'est ni un tert.-butyle ni un 1-Méthyl-3- (2,6,6-triméthyl-3-oxo-1-cyclohexèn-1-yl)-2-propényle,
lorsque $R_9$ est un 2-Butyle , $R_{10}$ n'est pas un 2-Butyle,
lorsque $R_9$ est un 3-butényle, $R_{10}$ n'est pas un 3-butényle,
lorsque $R_9$ est un Cyclopentyle. $R_{10}$ n'est pas un Cyclopentyle,
lorsque $R_9$ est un Cyclohexyle, $R_{10}$ n'est pas un Cyclohexyle,
lorsque $R_9$ est un 2-Méthyl-2-butyle, $R_{10}$ n'est pas un 2-Méthyl-2-butyle,
lorsque $R_9$ est un Benzyle, $R_{10}$ n'est pas un Benzyle,
lorsque $R_9$ est un 2-Benzyl-2-propyle, $R_{10}$ n'est pas un 2-Benzyl-2-propyle,
lorsque $R_9$ est un 1-Adamantyle, $R_{10}$ n'est pas un 1-Adamantyle,
lorsque $R_9$ est un 1-Diméthyl-1-benzyl-éthyle, $R_{10}$ n'est pas un 1-Diméthyl-1-benzyl-éthyle,
lorsque $R_9$ est un méthyle, $R_{10}$ n'est pas un tert-butyle,
lorsque $R_9$ est un Isobutyle $R_{10}$ n'est pas un tert.-butyle,
lorsque $R_9$ est un éthyle. $R_{10}$ n'est pas un tert.-butyle,
lorsque $R_9$ est un Undécafluoro-2-pentyle, $R_{10}$ n'est pas un Undécafluoro-2-pentyle,
lorsque $R_9$ est un p-tert.-butyl-benzyle, $R_{10}$ n'est pas un p-tert.-butyl-benzyle,
lorsque $R_9$ est un éthyle, $R_{10}$ n'est pas un 3,3,7-Triméthyl-cyclohepta-1,4,6-triényle,
lorsque $R_9$ est un méthyle, $R_{10}$ n'est pas un méthyle,
lorsque $R_9$ est un éthyle, $R_{10}$ n'est pas un éthyle,
lorsque $R_9$ est un n-propyle, $R_{10}$ n'est pas un n-propyle,
lorsque $R_9$ est un isobutyle, $R_{10}$ n'est pas un isobutyle,
lorsque $R_9$ est un allyle, $R_{10}$ n'est pas un allyle,
lorsque $R_9$ est un n-butyle, $R_{10}$ n'est pas un n-butyle,
lorsque $R_9$ est un tert.-amyle, $R_{10}$ n'est pas un tert.-amyle,
lorsque $R_9$ est un 2-Éthyl-hexyle, $R_{10}$ n'est pas un 2-Éthyl-hexyle,
lorsque $R_9$ est un 4-tert-Butyl-cyclohexyle, $R_{10}$ n'est pas un 4-tert-Butyl-cyclohexyle,
lorsque $R_9$ est un éthyle, $R_{10}$ n'est pas un 2-Méthyl-2-butyle,

lorsque $R_9$ est un Allyle $R_{10}$ n'est pas un tert.-butyle,
lorsque $R_9$ est un méthyle, $R_{10}$ n'est pas un Isopropyle,
lorsque $R_9$ est un éthyle, $R_{10}$ n'est pas un Isopropyle, et
lorsque $R_9$ est un éthyle, $R_{10}$ n'est pas un 2-butyle.

**2.** Utilisation d'un diester d'acide pyrocarbonique selon la revendication 1 pour la préparation de précurseurs de pigments solubles.

**3.** Utilisation selon la revendication 2, dans laquelle le précurseur de pigment est un composé de formule (IX),

$$A(B)_x \qquad (IX)$$

dans laquelle

x est un nombre entier compris entre 1 et 4
A est le reste d'un chromophore du groupe de la quinacridone, de l'anthraquinone, de l'indanthrone, du pérylène, de l'indigo, de la quinophtalone, de l'isoindolinone, de l'isoindoline, de la dioxazine, azoïque, de la phtalocyanine ou du dicétopyrrolo-pyrrole, lequel contient x atomes d'azote conjugués ou voisins avec au moins un groupe carbonyle, et B est un groupe lié avec un des ces atomes d'azote, de formule (X)

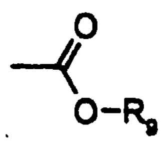
(X)

dans laquelle $R_9$ a la signification indiquée à la revendication 1.

**4.** Diesters d'acide pyrocarbonique selon la revendication 1, dans lesquels il s'agit du dicarbonate de di-3-éthyl-3-pentyle, du dicarbonate de di-2-méthyl-2-hexyle, du dicarbonate de di-2-benzyl-2-propyle, du dicarbonate de di-9-méthyl-9-octadécyle, du dicarbonate de di-2-méthyl-2-décyle, du dicarbonate de di-2-méthyl-2-nonadécyle, du dicarbonate de di-5-nonyle, du dicarbonate de di-(2-méthyl-3-butèn-1-yle) ou du dicarbonate de di-(2-méthyl-3-butyn-2-yle).